# EUROPEAN PATENT APPLICATION

(11) **EP 4 595 959 A1**
(43) Date of publication of application: **06.08.2025**
(21) Application number: 23872568.3
(22) Date of filing: 28.09.2023
(51) Int. Cl.: A61K 31/353, A61K 9/10, A61K 47/26, A61K 47/34, A61P 27/04

(54) **STERILIZED HETEROCYCLIDENE-ACETAMIDE-DERIVATIVE-CONTAINING SUSPENSION**

(30) Priority: 29.09.2022 JP 2022156060
(71) Applicant: Senju Pharmaceutical Co., Ltd., Osaka-shi, Osaka 541-0048 (JP); MOCHIDA PHARMACEUTICAL CO., LTD., Tokyo 160-8515 (JP)
(72) Inventor: YAMASHITA, Yuki, Osaka-shi, Osaka 541-0048 (JP)
(74) Representative: Engelhard, Markus
(86) International application number: PCT/JP2023/035547
(87) International publication number: WO 2024/071348

(57) **Abstract**

The present disclosure provides a suspension ophthalmic solution having exceptional re-dispersibility. More specifically, the present disclosure provides an aqueous suspension containing heat-treated (E)-2-(7-trifluoromethylchroman-4-yldene)-N-(7-hydroxy-5,6,7,8-tetrahydronaphthalen-1-yl)acetamide or a pharmacologically acceptable salt or solvate thereof. In particular, the present disclosure relates to technology for improving the re-dispersibility of a suspension that contains a heterocyclidene acetamide derivative or to formulation technology based thereupon.

## Description

### [Technical Field]

The present disclosure relates to the fields of pharmaceuticals, health care, biology, biotechnology, or the like. In particular, the present disclosure relates to a technique for improving redispersibility of a suspension liquid containing a heterocyclidene acetamide derivative; and a formulation technique based thereon.

### [Background Art]

The number of dry eye patients in Japan is estimated to be at least about 8 million, or about 22 million including potential patients who use over-the-counter eye drops without going to the hospital. It is said that there are 1 billion or more dry eye patients worldwide. It has been widely known that in modern society, use of televisions, computers, mobile terminals, etc. causes people to stare at screens more frequently to thereby reduce eye blink frequency, and use of air conditioners, etc. dries out the air, resulting in increased evaporation of tear fluid and thus a dry eye. Refractive surgery and use of contact lenses also result in a dry eye. Symptoms associated with the dry eye include, for example, ocular discomfort, dryness, burning, and irritation of an ocular surface.

When the dry eye occurs, the above symptoms appear as subjective symptoms, and their treatment requires regular eye drops over a long period of time. For this reason, most of the dry eye therapeutic drugs currently on the market are generally frequent-dosing types. For example, typically, a DIQUAS (registered trademark) eye drop is required to be applied 6 times a day, a HYALEIN (registered trademark) eye drop is required to be applied 5 to 6 times a day, and a MUCOSTA (registered trademark) eye drop is required to be applied 4 times a day.

A composition including (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof, or use thereof for treating a dry eye has been disclosed (PTL 1).

### [Citation List]

### [Patent Literature]

[PTL 1]
International Publication No. WO 2021/066144

### [Summary of Invention]

### [Solution to Problem]

The present disclosure provides an aqueous suspension liquid formulation with excellent redispersibility, the formation including (E)-2-(7-trifluoromethylchroman-4-ylidene)-N-(7-hydroxy-5,6,7,8-tetrahydronaphthalen-1-yl)acetamide, one of heterocyclidene acetamide derivatives, or a pharmaceutically acceptable salt or solvate thereof which has been subjected to a heat treatment.

Therefore, the present disclosure provides the following items.

### (Item 1)

An aqueous suspension liquid formulation including (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof which has been subjected to a heat treatment.

### (Item 1-1)

The aqueous suspension liquid formulation according to the above item, in which the heat treatment is a dry heat treatment.

### (Item 2)

The aqueous suspension liquid formulation according to the above item, in which the heat treatment is a treatment in a sterilization step.

### (Item 2-1)

The aqueous suspension liquid formulation according to the above item, in which the treatment in the sterilization step is a dry heat sterilization treatment.

### (Item 3)

The aqueous suspension liquid formulation according to any one of the above items, further including a nonionic surfactant.

### (Item 4)

The aqueous suspension liquid formulation according to any one of the above items, in which the heat treatment includes a heat treatment at a temperature of less than about 180 °C.

### (Item 5)

The aqueous suspension liquid formulation according to any one of the above items, in which the heat treatment includes a heat treatment at about 100 °C to about 175 °C.

### (Item 6)

The aqueous suspension liquid formulation according to any one of the above items, in which the heat treatment includes a heat treatment at about 100 °C to about 170 °C.

### (Item 7)

The aqueous suspension liquid formulation according to any one of the above items, in which the heat treatment includes a heat treatment at about 150 °C to about 170 °C.

### (Item 8)

The aqueous suspension liquid formulation according to any one of the above items, in which the heat treatment is performed for about 30 minutes to about 5 hours.

### (Item 9)

The aqueous suspension liquid formulation according to any one of the above items, in which the nonionic surfactant has a concentration of about 0.0001 w/v% to about 1 w/v% in the aqueous suspension liquid formulation.

### (Item 10)

The aqueous suspension liquid formulation according to any one of the above items, in which the nonionic surfactant has a concentration of about 0.001 w/v% to about 0.5 w/v% in the aqueous suspension liquid formulation.

### (Item 11)

The aqueous suspension liquid formulation according to any one of the above items, in which the nonionic surfactant has a concentration of about 0.01 w/v% to about 0.05 w/v% in the aqueous suspension liquid formulation.

### (Item 12)

The aqueous suspension liquid formulation according to any one of the above items, in which the nonionic surfactant is at least one selected from the group consisting of tyloxapol, polysorbate, polyethylene glycol monostearate, and a polyoxyethylene hydrogenated castor oil.

### (Item 13)

The aqueous suspension liquid formulation according to any one of the above items, in which the nonionic surfactant is tyloxapol.

### (Item 14)

The aqueous suspension liquid formulation according to any one of the above items, in which the (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof at a concentration of about 0.01 w/v% to about 5 w/v% in the aqueous suspension liquid formulation.

### (Item 15)

The aqueous suspension liquid formulation according to any one of the above items, in which the (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof has a concentration of about 0.3 w/v% to about 1 w/v% in the aqueous suspension liquid formulation.

### (Item 16)

An aqueous suspension liquid formulation including:
(E)-2-(7-trifluoromethylchroman-4-ylidene)-N-(7-hydroxy-5,6,7,8-tetrahydronaphthalen-1-yl)acetamide or a pharmaceutically acceptable salt or solvate thereof which has been subjected to a heat treatment; and
a nonionic surfactant,
the heat treatment including a heat treatment at a temperature of less than about 180 °C,
the heat treatment being performed for about 30 minutes to about 5 hours,
the (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof having a concentration of about 0.01 w/v% to about 5 w/v% in the aqueous suspension liquid formulation, and
the nonionic surfactant having a concentration of about 0.01 w/v% to about 0.05 w/v% in the aqueous suspension liquid formulation.

### (Item 17)

The aqueous suspension liquid formulation according to any one of the above items, the (E)-2-(7-trifluoromethylchroman-4-ylidene)-N-(7-hydroxy-5,6,7,8-tetrahydronaphthalen-1-yl)acetamide is (E)-2-(7-trifluoromethylchroman-4-ylidene)-N-((7R)-7-hydroxy-5,6,7,8-tetrahydronaphthalen-1-yl)acetamide.

### (Item 18)

The aqueous suspension liquid formulation according to any one of the above items, in which the aqueous suspension liquid formulation including (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof which has been subjected to a heat treatment, suspended particles of the (E)-2-(7-trifluoromethylchroman-4-ylidene)-N-(7-hydroxy-5,6,7,8-tetrahydronaphthalen-1-yl)acetamide or the pharmaceutically acceptable salt or solvate thereof being redispersed in the aqueous suspension liquid formulation in less than about 35 shakes as evaluated by a shaking operation.

### (Item 19)

The aqueous suspension liquid formulation according to any one of the above items, in which the aqueous suspension liquid formulation including (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof which has been subjected to a heat treatment, suspended particles of the (E)-2-(7-trifluoromethylchroman-4-ylidene)-N-(7-hydroxy-5,6,7,8-tetrahydronaphthalen-1-yl)acetamide or the pharmaceutically acceptable salt or solvate thereof being redispersed in the aqueous suspension liquid formulation in less than about 50 inversions as evaluated by an inversion operation.

### (Item 20)

An aqueous suspension liquid formulation including
(E)-2-(7-trifluoromethylchroman-4-ylidene)-N-(7-hydroxy-5,6,7,8-tetrahydronaphthalen-1-yl)acetamide or a pharmaceutically acceptable salt or solvate thereof which has been subjected to a heat treatment, the (E)-2-(7-trifluoromethylchroman-4-ylidene)-N-(7-hydroxy-5,6,7,8-tetrahydronaphthalen-1-yl)acetamide or the pharmaceutically acceptable salt or solvate thereof having improved redispersibility in the aqueous suspension liquid formulation.

### (Item 21)

The aqueous suspension liquid formulation according to any one of the above items, in which the improved redispersibility is a property of redispersing the suspended particles in less than about 35 shakes as evaluated by a shaking operation.

### (Item 22)

The aqueous suspension liquid formulation according to any one of the above items, in which the improved redispersibility is a property of redispersing the suspended particles in less than about 50 inversions as evaluated by an inversion operation.

### (Item 23)

An aqueous suspension liquid formulation including
(E)-2-(7-trifluoromethylchroman-4-ylidene)-N-(7-hydroxy-5,6,7,8-tetrahydronaphthalen-1-yl)acetamide or a pharmaceutically acceptable salt or solvate thereof which has been subjected to a heat treatment, the (E)-2-(7-trifluoromethylchroman-4-ylidene)-N-(7-hydroxy-5,6,7,8-tetrahydronaphthalen-1-yl)acetamide or the pharmaceutically acceptable salt or solvate thereof having an average particle size (D50) of about 1 µm to about 10 µm in the aqueous suspension liquid formulation.

### (Item 24)

A method for producing an aqueous suspension liquid formulation, the method including:
heat-treating (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof; and
mixing the thus-heat-treated (E)-2-(7-trifluoromethylchroman-4-ylidene)-N-(7-hydroxy-5,6,7,8-tetrahydronaphthalen-1-yl)acetamide or the pharmaceutically acceptable salt or solvate thereof with a solvent.

### (Item 25)

(E)-2-(7-trifluoromethylchroman-4-ylidene)-N-(7-hydroxy-5,6,7,8-tetrahydronaphthalen-1-yl)acetamide or a pharmaceutically acceptable salt or solvate thereof which has been subjected to a heat treatment.

### (Item 26)

The aqueous suspension liquid formulation according to any one of the above items, in which the aqueous suspension liquid formulation has a pH of about 4 to about 8.

### (Item 27)

The aqueous suspension liquid formulation according to any one of the above items, in which the aqueous suspension liquid formulation is contained in a plastic container.

### (Item 28)

The aqueous suspension liquid formulation according to any one of the above items, in which the plastic container is made of polyethylene or polypropylene.

### (Item B1)

An aqueous suspension liquid formulation including
(E)-2-(7-trifluoromethylchroman-4-ylidene)-N-(7-hydroxy-5,6,7,8-tetrahydronaphthalen-1-yl)acetamide or a pharmaceutically acceptable salt or solvate thereof which has been subjected to a gamma sterilization treatment.

### (Item B2)

The aqueous suspension liquid formulation according to any one of the above items, further including a nonionic surfactant.

### (Item B3)

An aqueous suspension liquid formulation including:
(E)-2-(7-trifluoromethylchroman-4-ylidene)-N-(7-hydroxy-5,6,7,8-tetrahydronaphthalen-1-yl)acetamide or a pharmaceutically acceptable salt or solvate thereof which has been subjected to a gamma sterilization treatment; and
a nonionic surfactant.

### (Item B4)

The aqueous suspension liquid formulation according to any one of the above items, in which the gamma sterilization treatment includes a gamma treatment at about 10 to about 50 kGy.

### (Item B5)

The aqueous suspension liquid formulation according to any one of the above items, in which the gamma sterilization treatment includes a gamma treatment at about 25 kGy.

### (Item B6)

The aqueous suspension liquid formulation according to any one of the above items, in which the nonionic surfactant has a concentration of about 0.0001 w/v% to about 0.1 w/v% in the aqueous suspension liquid formulation.

### (Item B7)

The aqueous suspension liquid formulation according to any one of the above items, in which the nonionic surfactant has a concentration of about 0.001 w/v% to about 0.5 w/v% in the aqueous suspension liquid formulation.

### (Item B8)

The aqueous suspension liquid formulation according to any one of the above items, in which the nonionic surfactant has a concentration of about 0.01 w/v% to about 0.05 w/v% in the aqueous suspension liquid formulation.

### (Item B9)

The aqueous suspension liquid formulation according to any one of the above items, in which the nonionic surfactant is at least one selected from the group consisting of tyloxapol, polysorbate, polyethylene glycol monostearate, and a polyoxyethylene hydrogenated castor oil.

### (Item B10)

The aqueous suspension liquid formulation according to any one of the above items, in which the nonionic surfactant is tyloxapol.

### (Item B11)

The aqueous suspension liquid formulation according to any one of the above items, in which the (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof has a concentration of about 0.01 w/v% to about 5 w/v% in the aqueous suspension liquid formulation.

### (Item B12)

The aqueous suspension liquid formulation according to any one of the above items, in which the (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof has a concentration of about 0.3 w/v% to about 1 w/v% in the aqueous suspension liquid formulation.

### (Item B13)

An aqueous suspension liquid formulation including:
(E)-2-(7-trifluoromethylchroman-4-ylidene)-N-(7-hydroxy-5,6,7,8-tetrahydronaphthalen-1-yl)acetamide or a pharmaceutically acceptable salt or solvate thereof which has been subjected to a gamma sterilization treatment; and
a nonionic surfactant,
the gamma sterilization treatment including a gamma treatment at about 10 to about 50 kGy,
the (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof having a concentration of about 0.01 w/v% to about 5 w/v% in the aqueous suspension liquid formulation, and
the nonionic surfactant having a concentration of about 0.01 w/v% to about 0.05 w/v% in the aqueous suspension liquid formulation.

### (Item B14)

The aqueous suspension liquid formulation according to any one of the above items, the (E)-2-(7-trifluoromethylchroman-4-ylidene)-N-(7-hydroxy-5,6,7,8-tetrahydronaphthalen-1-yl)acetamide is (E)-2-(7-trifluoromethylchroman-4-ylidene)-N-((7R)-7-hydroxy-5,6,7,8-tetrahydronaphthalen-1-yl)acetamide.

### (Item B15)

The aqueous suspension liquid formulation according to any one of the above items, the aqueous suspension liquid formulation including (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof which has been subjected to a gamma sterilization treatment, suspended particles of the (E)-2-(7-trifluoromethylchroman-4-ylidene)-N-(7-hydroxy-5,6,7,8-tetrahydronaphthalen-1-yl)acetamide or the pharmaceutically acceptable salt or solvate thereof being redispersed in the aqueous suspension liquid formulation in less than about 35 shakes as evaluated by a shaking operation.

### (Item B16)

The aqueous suspension liquid formulation according to any one of the above items, including (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof which has been subjected to a gamma sterilization treatment, suspended particles of the (E)-2-(7-trifluoromethylchroman-4-ylidene)-N-(7-hydroxy-5,6,7,8-tetrahydronaphthalen-1-yl)acetamide or the pharmaceutically acceptable salt or solvate thereof being redispersed in the aqueous suspension liquid formulation in less than about 50 inversions as evaluated by an inversion operation.

### (Item B17)

An aqueous suspension liquid formulation including
(E)-2-(7-trifluoromethylchroman-4-ylidene)-N-(7-hydroxy-5,6,7,8-tetrahydronaphthalen-1-yl)acetamide or a pharmaceutically acceptable salt or solvate thereof which has been subjected to a gamma sterilization treatment, the (E)-2-(7-trifluoromethylchroman-4-ylidene)-N-(7-hydroxy-5,6,7,8-tetrahydronaphthalen-1-yl)acetamide or the pharmaceutically acceptable salt or solvate thereof having improved redispersibility in the aqueous suspension liquid formulation.

### (Item B18)

The aqueous suspension liquid formulation according to any one of the above items, in which the improved redispersibility is a property of redispersing the suspended particles in about 35 shakes or less as evaluated by a shaking operation.

### (Item B19)

The aqueous suspension liquid formulation according to any one of the above items, in which the improved redispersibility is a property of redispersing the suspended particles in about 50 inversions or less as evaluated by an inversion operation.

### (Item B20)

(E)-2-(7-trifluoromethylchroman-4-ylidene)-N-(7-hydroxy-5,6,7,8-tetrahydronaphthalen-1-yl)acetamide or a pharmaceutically acceptable salt or solvate thereof which has been subjected to a gamma sterilization treatment.

### (Item B21)

The aqueous suspension liquid formulation according to any one of the above items, in which the aqueous suspension liquid formulation has a pH of about 4 to about 8.

### (Item B22)

The aqueous suspension liquid formulation according to any one of the above items, in which the aqueous suspension liquid formulation is contained in a plastic container.

### (Item B23)

The aqueous suspension liquid formulation according to any one of the above items, in which the plastic container is made of polyethylene or polypropylene.

### (Item A1)

A method for producing an aqueous suspension liquid formulation, the method including:
heat-treating (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof; and
mixing the thus-heat-treated (E)-2-(7-trifluoromethylchroman-4-ylidene)-N-(7-hydroxy-5,6,7,8-tetrahydronaphthalen-1-yl)acetamide or the pharmaceutically acceptable salt or solvate thereof with a solvent.

### (Item A1-1)

The method according to any one of the above items, in which the heat treatment is a dry heat treatment.

### (Item A2)

The method according to any one of the above items, in which the heat treatment is a treatment in a sterilization step.

### (Item A2-2)

The method according to any one of the above items, in which the treatment in the sterilization step is a dry heat sterilization treatment.

### (Item A3)

The method according to any one of the above items, further mixing with a nonionic surfactant.

### (Item A4)

The method according to any one of the above items, in which the heat treatment includes a heat treatment at a temperature of less than about 180 °C.

### (Item A5)

The method according to any one of the above items, in which the heat treatment includes a heat treatment at about 100 °C to about 175 °C.

### (Item A6)

The method according to any one of the above items, in which the heat treatment includes a heat treatment at about 100 °C to about 170 °C.

### (Item A7)

The method according to any one of the above items, in which the heat treatment includes a heat treatment at about 150 °C to about 170 °C.

### (Item A8)

The method according to any one of the above items, in which the heat treatment is performed for about 30 minutes to about 5 hours.

### (Item A9)

The method according to any one of the above items, in which the nonionic surfactant has a concentration of about 0.0001 w/v% to about 1 w/v% in the aqueous suspension liquid formulation.

### (Item A10)

The method according to any one of the above items, in which the nonionic surfactant has a concentration of about 0.001 w/v% to about 0.5 w/v% in the aqueous suspension liquid formulation.

### (Item A11)

The method according to any one of the above items, in which the nonionic surfactant has a concentration of about 0.01 w/v% to about 0.05 w/v% in the aqueous suspension liquid formulation.

### (Item A12)

The method according to any one of the above items, in which the nonionic surfactant is at least one selected from the group consisting of tyloxapol, polysorbate, polyethylene glycol monostearate, and a polyoxyethylene hydrogenated castor oil.

### (Item A13)

The method according to any one of the above items, in which the nonionic surfactant is tyloxapol.

### (Item A14)

The method according to any one of the above items, in which the (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof has a concentration of about 0.01 w/v% to about 5 w/v% in the aqueous suspension liquid formulation.

### (Item A15)

The method according to any one of the above items, in which the (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof has a concentration of about 0.3 w/v% to about 1 w/v% in the aqueous suspension liquid formulation.

### (Item A16)

The method according to any one of the above items, the (E)-2-(7-trifluoromethylchroman-4-ylidene)-N-(7-hydroxy-5,6,7,8-tetrahydronaphthalen-1-yl)acetamide is (E)-2-(7-trifluoromethylchroman-4-ylidene)-N-((7R)-7-hydroxy-5,6,7,8-tetrahydronaphthalen-1-yl)acetamide.

### (Item A17)

The method according to any one of the above items, in which the aqueous suspension liquid formulation has a pH of about 4 to about 8.

### (Item A18)

The method according to any one of the above items, in which suspended particles of the heat-treated (E)-2-(7-trifluoromethylchroman-4-ylidene)-N-(7-hydroxy-5,6,7,8-tetrahydronaphthalen-1-yl)acetamide or the pharmaceutically acceptable salt or solvate thereof are redispersed in the aqueous suspension liquid formulation in less than about 35 shakes as evaluated by a shaking operation.

### (Item A19)

The method according to any one of the above items, in which suspended particles of the heat-treated (E)-2-(7-trifluoromethylchroman-4-ylidene)-N-(7-hydroxy-5,6,7,8-tetrahydronaphthalen-1-yl)acetamide or the pharmaceutically acceptable salt or solvate thereof are redispersed in the aqueous suspension liquid formulation in less than about 50 inversions as evaluated by an inversion operation.

### (Item A20)

The method according to any one of the above items, in which the (E)-2-(7-trifluoromethylchroman-4-ylidene)-N-(7-hydroxy-5,6,7,8-tetrahydronaphthalen-1-yl)acetamide or the pharmaceutically acceptable salt or solvate thereof has improved redispersibility in the aqueous suspension liquid formulation.

### (Item A21)

The method according to any one of the above items, in which the improved redispersibility is a property of redispersing the suspended particles in about 35 shakes or less as evaluated by a shaking operation.

### (Item A22)

The method according to any one of the above items, in which the improved redispersibility is a property of redispersing the suspended particles in about 50 inversions or less as evaluated by an inversion operation.

### (Item A23)

The method according to any one of the above items, in which the (E)-2-(7-trifluoromethylchroman-4-ylidene)-N-(7-hydroxy-5,6,7,8-tetrahydronaphthalen-1-yl)acetamide or the pharmaceutically acceptable salt or solvate thereof has an average particle size of about 1 µm to about 10 µm in the aqueous suspension liquid formulation.

### (Item A24)

The method according to any one of the above items, in which the aqueous suspension liquid formulation is contained in a plastic container.

### (Item A25)

The method according to any one of the above items, in which the plastic container is made of polyethylene or polypropylene.

### (Item C1)

A method for producing an aqueous suspension liquid formulation, the method including:
gamma-treating (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof; and
mixing the thus-gamma-treated (E)-2-(7-trifluoromethylchroman-4-ylidene)-N-(7-hydroxy-5,6,7,8-tetrahydronaphthalen-1-yl)acetamide or the pharmaceutically acceptable salt or solvate thereof with a solvent.

### [Advantageous Effects of Invention]

The present disclosure can provide an aqueous suspension liquid formulation with excellent redispersibility.

By providing the above features, the present disclosure suppresses a phenomenon that suspended particles are not uniformly dispersed, enables stable administration of a required amount of an active ingredient and realizes of a stable and sufficient exertion of pharmacological effect. This contributes to improvement of patient adherence and convenience.

### [Description of Embodiments]

The present disclosure will be described. It should be understood that throughout this specification, singular expressions also include the concept of their plural forms, unless otherwise noted. Thus, it should be understood that singular articles (e.g., "a", "an", "the", etc. in English) also include the concept of their plural forms, unless otherwise noted. It should also be understood that terms used herein are to be used in the sense normally used in the art, unless otherwise noted. Therefore, unless otherwise defined, all technical and scientific terms used herein have the same meaning as commonly understood by those skilled in the art to which the present disclosure pertains. In the event of a conflict, this specification (including definitions) shall prevail.

### (Definitions)

As used herein, the term "about" means ± 10% of the numeral value that follows, unless otherwise noted.

As used herein, the term "or" is used when "at least one or more" of matters enumerated in a text can be employed. The same is true of "alternatively". When the phrase "within a range" of "two values" is specified herein, the range includes the two values themselves.

As used herein, the phrase "aqueous suspension liquid formulation" is used with the same meaning as the term commonly used in the art and refers to a liquid formulation that contains water in at least a portion thereof, in which components to be mixed are in suspension and in which solid particles are present in the liquid. Since (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof has extremely less soluble in water, the (E)-2-(7-trifluoromethylchroman-4-ylidene)-N-(7-hydroxy-5,6,7,8-tetrahydronaphthalen-1-yl)acetamide or the pharmaceutically acceptable salt or solvate thereof becomes suspended particles, but partially dissolved in the aqueous suspension liquid formulation of the present disclosure.

As used herein, the phrase "pharmaceutically acceptable salt" means a relatively non-toxic, inorganic or organic acid addition salt or inorganic or organic base addition salt of a compound of the present disclosure.

As used herein, the term "solvate" means one aggregate formed by interaction of a compound of the present disclosure or a pharmaceutically acceptable salt thereof with any solvent, and includes, for example, a solvate with an organic solvent (e.g., an alcoholate (ethanolate, etc.)) and a hydrate. When the hydrate is formed, the compound of the present disclosure or the pharmaceutically acceptable salt thereof may be coordinated with any number of water molecules. The hydrate may include a monohydrate and a dihydrate and the like.

As used herein, the term "redispersibility" refers to ease of uniform dispersion of particles (also referred to as "suspended particles" in the case of a suspension liquid) that have settled in a solvent of a liquid containing the particles, such as a suspension liquid, throughout the liquid again after the liquid is stored for a certain period of time. As used herein, the term "redispersibility" is evaluated by testing with a shaking or inversion operation.

As used herein, the phrase "shaking operation" refers to an action of holding a container filled with the "aqueous suspension liquid formulation" in the hand and shaking it up and down. For the "shaking operation", the container is shaken down 10 to 15 cm vertically and then shaken up to its original position, which is defined as one shake. Five shakes are determined as one set and the contained is shaken at a rate of 1.1 seconds/set. A test for the redispersibility with the "shaking operation" is performed on three samples per formulation, and an average number of sets is calculated from the number of sets required for each sample to redisperse, which is then converted to the number of shakes.

As used herein, the phrase "inversion operation" refers to an action of holding a container filled with the "aqueous suspension liquid formulation" in the hand and turning it up and down. A test for the redispersibility with the "inversion operation" is performed on three samples per formulation. The container is turned 180° vertically and then turned 180° again to stand upright at a rate of 1 second/inversion, which is defined as one inversion.

As used herein, the phrase "improved redispersibility" means that particles that have settled in a liquid containing the particles, such as a suspension liquid, are easier to disperse again, and if the number of actions of the above "shaking operation" or "inversion operation" is reduced in comparison for any formulations, the redispersibility can be said to be improved.

As used herein, the phrase "average particle size of suspended particles" refers to a median diameter (D₅₀) of particle diameters of particles of (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof and is measured by a laser diffraction particle size distribution analyzer.

As used herein, the phrase "nonionic surfactant" is also referred to as a non-ion surfactant, and refers to a surfactant of which hydrophilic group portion is nonionic. Those skilled in the art can easily determine whether a compound is a nonionic surfactant or not by checking that the compound does not ionize (show ionicity) when dissolved in water. Examples of the nonionic surfactant include tyloxapol, polyoxyl stearates (polyethylene glycol monostearate, polyethylene glycol monostearate 40 (MYS-40), polyethylene glycol monostearate 400, etc.), polyoxyethylene sorbitan fatty acid esters (polyoxyethylene sorbitan monooleate (polysorbate 80), polyoxyethylene sorbitan monolaurate, polyoxyethylene sorbitan monopalmitate, polyoxyethylene sorbitan monostearate, polyoxyethylene sorbitan tristearate, etc.), and polyoxyethylene hydrogenated castor oils (polyoxyethylene hydrogenated castor oil 10, polyoxyethylene hydrogenated castor oil 40, polyoxyethylene hydrogenated castor oil 50, polyoxyethylene hydrogenated castor oil 60 (HCO-60), etc.).

As used herein, the phrase "heat treatment" refers to application of heat to a substance, molecule, aqueous suspension liquid formulation, or composition of interest and it only has to be substantially heated. The "heat treatment" includes, for example, a dry heat treatment, a wet heat treatment, and a high-pressure steam treatment. A temperature of the heat treatment may mean a temperature of the substance, molecule, aqueous suspension liquid formulation, or composition of interest, or it may be a temperature set on a device to be used for application of heat or a temperature shown on a display. A period of time of the heat treatment may mean a period of time during which heat is applied to a substance, molecule, aqueous suspension liquid formulation, or composition of interest to reach a predetermined temperature and the predetermined temperature is maintained as determined by an average temperature, or it may be a period of time set on a device to be used for application of heat or a period of time shown on a display. In the present disclosure, the heat treatment (e.g., dry heat treatment, wet heat treatment, or high-pressure steam treatment) is performed on (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof itself. The dry heat treatment may be performed on powder of (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof. The term "powder" means a powder or granule (which is made of solid particles with any particle size distribution and in which individual particles are interacted with each other by, for example, an electrostatic force and a van der Waals force).

As used herein, the term "sterilization" or the phrase "sterilization treatment" means substantial removal, disinfection, or deactivation, etc., of a virus, a microorganism, etc., present in a substance, molecule, aqueous suspension liquid formulation, or composition of interest. The sterilization may include, for example, a gamma sterilization treatment, a dry heat sterilization, and an autoclave sterilization. Thus, the phrase "gamma sterilization treatment" refers to sterilization of a substance, molecule, aqueous suspension liquid formulation, or composition of interest by irradiating the substance, molecule, or the like with gamma rays. In addition, the phrase "dry heat sterilization" refers to a sterilization treatment by heating in a dry state. In the present disclosure, the sterilization or the sterilization treatment is performed on (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof itself. The gamma sterilization treatment may be performed on powder of (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof.

As used herein, the term "solvent" is used to encompass water, an organic solvent, and a mixture of water and an organic solvent, which may be filter-sterilized or otherwise sterilized. The thus-sterilized solvent is also included in the "solvent" herein. In addition to the water, the organic solvent, or the mixture of water and an organic solvent, the "solvent" may also contain any additive such as a viscous agent, a stabilizer, a pH adjuster, a buffer, and a preservative (antiseptic) dissolved therein. Such an additive-containing solvent is also included in the "solvent" herein.

### (Preferred Embodiment)

Preferred embodiments of the present disclosure will be described. Embodiments provided below are given for a better understanding of the present disclosure and the scope of the present disclosure should not be limited to the following description. Therefore, it is clear that those skilled in the art may make modifications as appropriate within the scope of the present disclosure, taking into account the description herein. The following embodiments of the present disclosure may also be used alone or in combination.

### (Aqueous suspension liquid formulation)

In one aspect of the present disclosure, an aqueous suspension liquid formulation including (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof which has been subjected to a heat treatment can be provided.

In another aspect of the present disclosure, an aqueous suspension liquid formulation including (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof which has been subjected to a heat treatment; and a nonionic surfactant can be provided.

In one aspect of the present disclosure, an aqueous suspension liquid formulation including (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof which has been subjected to a heat treatment at about 150 °C to about 170 °C can be provided.

In another aspect of the present disclosure, an aqueous suspension liquid formulation including (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof which has been subjected to a heat treatment at about 150 °C to about 170 °C; and a nonionic surfactant can be provided.

In one aspect of the present disclosure, an aqueous suspension liquid formulation including (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof which has been subjected to a sterilization treatment by a heat treatment at about 100 °C to about 170 °C can be provided.

In another aspect of the present disclosure, an aqueous suspension liquid formulation including (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof which has been subjected to a sterilization treatment by a heat treatment at about 100 °C to about 170 °C; and a nonionic surfactant can be provided.

In one aspect of the present disclosure, an aqueous suspension liquid formulation including (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof which has been subjected to a gamma sterilization treatment can be provided.

In another aspect of the present disclosure, an aqueous suspension liquid formulation including (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof which has been subjected to a gamma sterilization treatment; and a nonionic surfactant can be provided.

In one aspect of the present disclosure, a method for producing an aqueous suspension liquid formulation, the method including heat-treating (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof; and mixing the thus-heat-treated (E)-2-(7-trifluoromethylchroman-4-ylidene)-N-(7-hydroxy-5,6,7,8-tetrahydronaphthalen-1-yl)acetamide or the pharmaceutically acceptable salt or solvate thereof with a solvent can be provided.

In another aspect of the present disclosure, a method for producing an aqueous suspension liquid formulation, the method including sterilizing (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof with gamma rays; and mixing the thus-sterilized (E)-2-(7-trifluoromethylchroman-4-ylidene)-N-(7-hydroxy-5,6,7,8-tetrahydronaphthalen-1-yl)acetamide or the pharmaceutically acceptable salt or solvate thereof with a solvent can be provided.

In one aspect of the present disclosure, (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof which has been subjected to a heat treatment can be provided.

In one aspect of the present disclosure, (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof which has been subjected to a heat treatment at about 150 °C to about 170 °C can be provided.

In one aspect of the present disclosure, (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof which has been subjected to a sterilization treatment by a heat treatment at about 100 °C to about 170 °C can be provided.

In another aspect of the present disclosure, (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof which has been subjected to a gamma sterilization treatment can be provided.

The (E)-2-(7-trifluoromethylchroman-4-ylidene)-N-(7-hydroxy-5,6,7,8-tetrahydronaphthalen-1-yl)acetamide includes a R isomer thereof (CAS. No. 920332-28-1), a S isomer thereof (CAS. No. 920332-29-2), or a racemate thereof (CAS. No. 920332-27-0), more preferably is the R isomer thereof ((E)-2-(7-trifluoromethylchroman-4-ylidene)-N-((7R)-7-hydroxy-5,6,7,8-tetrahydronaphthalen-1-yl)acetamide (herein also referred to as Compound (1)).

The pharmaceutically acceptable salt of the compound of the present disclosure is not particularly limited as long as it is a pharmaceutically acceptable salt. Specifical examples thereof include mineral acids such as hydrochloric acid, hydrobromic acid, hydroiodic acid, sulfuric acid, nitric acid, phosphoric acid, etc.; organic carboxylic acids, for example, aliphatic monocarboxylic acids such as formic acid, acetic acid, propionic acid, butyric acid, valeric acid, enanthic acid, capric acid, myristic acid, palmitic acid, stearic acid, lactic acid, sorbic acid, mandelic acid, etc., aromatic monocarboxylic acids such as benzoic acid, salicylic acid, etc., aliphatic dicarboxylic acids such as oxalic acid, malonic acid, succinic acid, fumaric acid, maleic acid, malic acid, tartaric acid, etc. and aliphatic tricarboxylic acids such as citric acid, etc.; acid addition salts with organic sulfonic acids, for example, aliphatic sulfonic acids such as methanesulfonic acid, ethanesulfonic acid, 2-hydroxyethanesulfonic acid, etc. and aromatic sulfonic acids such as benzene sulfonic acid, p-toluenesulfonic acid, etc.; and inorganic base addition salts with metals, for example, alkali metals or alkaline earth metals such as sodium, potassium, magnesium, calcium, etc. and organic base addition salts such as methylamine, ethylamine, ethanolamine, pyridine, lysine, arginine, ornithine, etc.

These salts can be obtained by a routine procedure, e.g., by mixing equivalent amounts of the compound of the present disclosure with a solution containing a desired acid or base, and then filtering out a desired salt or collecting the desired salt by distilling a solvent off. The compound of the present disclosure or a salt thereof can also form a solvate with water or a solvent such as ethanol.

The (E)-2-(7-trifluoromethylchroman-4-ylidene)-N-(7-(-hydroxy-5,6,7,8-tetrahydronaphthalen-1-yl)acetamide has excellent antagonism against a transient receptor potential vanilloid 1 (hereinafter described as "TRPV1". The TRPV1 is also referred to as "vanilloid receptor 1 (VR1)").

The R isomer (Compound (1)), the S isomer, or the racemate of the (E)-2-(7-trifluoromethylchroman-4-ylidene)-N-(7-hydroxy-5,6,7,8-tetrahydronaphthalen-1-yl)acetamide is described in PCT International Publication No. WO2007/010383, Japanese Patent No. 4754566, Japanese Patent No. 6230743, PCT International Publication No. WO2018/221543, Japanese Patent No. 6830569, PCT International Publication No. WO2021/038889, and PCT International Publication No. WO2021/039023. The R isomer (Compound (1)), the S isomer, or the racemate can be produced by the production methods described in the above-mentioned patent documents. The contents of the above-mentioned patent documents are incorporated herein by reference in their entirety.

The TRPV1 is a TRP channel cloned from the dorsal root ganglion (DRG) as a cation channel responsive to capsaicin, is sensitive to heat of 43 °C or more and protons, and has been studied as a key nociceptive molecule (SEIKAGAKU Vol. 85, No. 7: 561-565). The TRPV1 is known to increase its activity and induce hyperalgesia upon inflammation and tissue injury. Therefore, the TRPV1 has attracted attention as a drug target candidate for use in a treatment of pain.

A TRPV1 antagonist has previously been reported to be effective in various pain models, including inflammatory pain, neuropathic pain, and osteoarthritis (SEIKAGAKU Vol. 85, No. 7: 561-565).

In one embodiment of the present disclosure, (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof is heat-treated, and the thus-heat-treated (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof is then mixed with a solvent to form an aqueous suspension liquid formulation. Thus, the (E)-2-(7-trifluoromethylchroman-4-ylidene)-N-(7-hydroxy-5,6,7,8-tetrahydronaphthalen-1-yl)acetamide or the pharmaceutically acceptable salt or solvate thereof can have improved redispersibility.

In another embodiment of the present disclosure, (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof is sterilized with gamma rays, and the thus-sterilized (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof is then mixed with a solvent form an aqueous suspension liquid formulation. Thus, the (E)-2-(7-trifluoromethylchroman-4-ylidene)-N-(7-hydroxy-5,6,7,8-tetrahydronaphthalen-1-yl)acetamide or the pharmaceutically acceptable salt or solvate thereof can have improved redispersibility.

In one embodiment of the present disclosure, (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof can present in an aqueous suspension liquid of the present disclosure at a concentration of typically about 0.01 w/v% to about 5 w/v%, preferably about 0.1 w/v% to about 3 w/v%, more preferably about 0.2 w/v% to about 2 w/v%, particularly preferably about 0.2 w/v% to about 1.5 w/v%, and further preferably about 0.3 w/v% to about 1.0 w/v%.

In one embodiment of the present disclosure, when the R isomer of (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof is used, it can present in an aqueous suspension liquid formulation of the present disclosure at a concentration of typically about 0.01 w/v% to about 5 w/v%, preferably about 0.1 w/v% to about 3 w/v%, more preferably about 0.2 w/v% to about 2 w/v%, particularly preferably about 0.2 w/v% to about 1.5 w/v%, and further preferably about 0.3 w/v% to about 1.0 w/v%.

In one embodiment of the present disclosure, (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof can be heat-treated as a treatment in a sterilization step of the (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof typically at a temperature of lower than about 180 °C, preferably about 175 °C or less, more preferably about 100 to about 175 °C, particularly about 100 to about 170 °C, further preferably about 120 to about 170 °C, even more preferably about 145 to about 175 °C, and most preferably about 150 to about 170 °C.

In one embodiment of the present disclosure, (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof is typically heat-treated for about 10 minutes to about 24 hours, more preferably for about 30 minutes to about 12 hours, particularly preferably for about 30 minutes to about 8 hours, and further preferably for about 30 minutes to about 5 hours.

In one embodiment of the present disclosure, (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof can be sterilized with gamma rays as a treatment in a sterilization step of the (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof typically at a dose of about 10 to about 50 kGy and further preferably about 25 kGy.

In one embodiment of the present disclosure, the nonionic surfactant is not particularly limited as long as it is pharmaceutically acceptable. Examples thereof include tyloxapol, polyoxyl stearates (polyethylene glycol monostearate, polyethylene glycol monostearate 40 (MYS-40), polyethylene glycol monostearate 400, etc.), polyoxyethylene sorbitan fatty acid esters (polyoxyethylene sorbitan monooleate (polysorbate 80), polyoxyethylene sorbitan monolaurate, polyoxyethylene sorbitan monopalmitate, polyoxyethylene sorbitan monostearate, polyoxyethylene sorbitan tristearate, etc.), and polyoxyethylene hydrogenated castor oils (polyoxyethylene hydrogenated castor oil 10, polyoxyethylene hydrogenated castor oil 40, polyoxyethylene hydrogenated castor oil 50, polyoxyethylene hydrogenated castor oil 60 (HCO 60), etc.). The nonionic surfactant may be used alone or two or more thereof may be used in combination. The nonionic surfactant is preferably tyloxapol, polysorbate, polyethylene glycol monostearate, or a polyoxyethylene hydrogenated castor oil, more preferably tyloxapol, polysorbate, or polyethylene glycol monostearate, and further preferably tyloxapol from the viewpoint of redispersibility or stability of the aqueous suspension liquid formulation.

In one embodiment of the present disclosure, the nonionic surfactant can be present in the aqueous suspension liquid formulation of the present disclosure at a concentration of about 0.0001 w/v% to about 1 w/v%, preferably about 0.0005 w/v% to about 0.5 w/v%, more preferably about 0.001 w/v% to about 0.5 w/v%, particularly preferably about 0.005 w/v% to about 0.1 w/v%, and further preferably about 0.01 w/v% to about 0.05 w/v%.

### (Redispersibility)

Since (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof is less soluble in water, particles thereof float on a water surface in the absence of a dispersant such as a nonionic surfactant, an ionic surfactant, or a water-soluble polymer, making it impossible to prepare an aqueous suspension liquid. In addition, if a dispersant other than a nonionic surfactant is used, it is essential to include the nonionic surfactant in the aqueous suspension liquid formulation because the dispersant needs to be included at a concentration substantially higher than a pharmaceutically acceptable concentration. The present disclosure can provide an aqueous suspension liquid formulation with improved redispersibility by heat-treating (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof in the presence of a nonionic surfactant as a dispersant.

In another aspect of the present disclosure, an aqueous suspension liquid formulation with improved redispersibility can be provided by sterilizing (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof with gamma rays.

In one embodiment, the redispersibility can be determined to be improved when the number of shakes required for the aforementioned redispersion is typically less than about 35, preferably about 30 or less, more preferably about 25 or less, and particularly preferably about 20 or less.

A measurement with the inversion operation can be said to reproduce evaluation when used by a patient who is weak-handed due to some disability or symptoms and has difficulty in redispersion by shaking, and thus can also be said to evaluate redispersibility when used by the patient or elderly people.

In one embodiment, the redispersibility can be determined to be improved when the number of inversion operations required for the aforementioned redispersion is typically less than about 50 times, preferably about 45 times or less.

In one embodiment, the redispersibility can also be evaluated with any means, for example, by filling a container with the aqueous suspension liquid of the present disclosure and shaking the container at any rate and/or any shaking width or inverting the container to thereby measure the number of times required for suspended particles to be redispersed.

In one embodiment, an average particle size (D₅₀) of (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof in the aqueous suspension liquid of the present disclosure is not particularly limited and is typically about 1 µm to about 10 µm, preferably about 0.5 µm to about 5 µm, particularly preferably about 2 µm to about 4 µm, further preferably about 2.3 µm to about 3.3 µm, and most preferably about 2.1 µm to about 3.3 µm. The average particle size falling within such a range can improve the redispersibility.

In one embodiment, (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof in the aqueous suspension liquid of the present disclosure can be used in a crystalline form, which is not particularly limited as long as it does not affect the redispersibility. For example, in the present disclosure, a type I crystal, a type II crystal, or a type III crystal of (E)-2-(7-trifluoromethylchroman-4-ylidene)-N-((7R)-7-hydroxy-5,6,7,8-tetrahydronaphthalene-1-yl)acetamide, or a mixture thereof disclosed in PCT International Publication No. WO 2018/221543 and Japanese Patent No. 6230743 can be used. Preferably, the type I crystal is used.

### (Dosage form)

In one embodiment of the present disclosure, the aqueous suspension liquid formulation is an ophthalmic suspension liquid formulation and can be provided as an ocular injection solution, an eye drop, or an ocular perfusate. For example, the ophthalmic suspension liquid formulation can be provided in a form of a suspension of an active ingredient in an aqueous solvent (e.g., phosphate buffered saline) or in a form of a solution of an active ingredient in an aqueous solvent.

In one embodiment of the present disclosure, the aqueous suspension liquid formulation can be an eye drop for treating a dry eye. The dry eye is a disease that is accompanied by subjective symptoms such as eye discomfort and requires a long-term and regular treatment. In addition, since a therapeutic drug for the dry eye is generally designed to be administered frequently, there is a strong need for a formulation with good patient adherence and convenience. Furthermore, there is concern that in the case of a suspension liquid with poor redispersibility, suspended particles may not be dispersed uniformly, a desired active ingredient may not be administered, and thus a pharmacological effect may not be sufficiently exerted. From these viewpoints, the redispersibility is a major issue in an eye drop for treating a dry eye, and provision of the aqueous suspension liquid formulation of the present disclosure with excellent redispersibility is highly valuable.

The aqueous suspension liquid formulation of the present disclosure can be administered by any suitable route determined by those skilled in the art and can be formulated to be suitable for administration via an administration route selected from, but not limited to, ocular injection, topical application (including application to the eye), instillation, intravenous injection, infusion, oral, parenteral, transdermal, etc.

### (Additive and/or excipient)

The aqueous suspension liquid formulation of the present disclosure may include any pharmaceutically acceptable additive and/or excipient known in the art. Examples of the additive include, but is not limited to, a viscous agent, a stabilizer, a pH adjuster, a buffer, and a preservative (antiseptic).

The viscous agent is not particularly limited to the extent that it is pharmaceutically acceptable. Examples thereof include a water-soluble polymer such as a carboxy vinyl polymer, polyvinyl pyrrolidone, polyethylene glycol, polyvinyl alcohol, xanthan gum, sodium chondroitin sulfate, sodium hyaluronate, etc.; and a cellulosic polymer such as hydroxyethyl cellulose, methyl cellulose, hydroxypropyl cellulose, hydroxypropyl methyl cellulose, sodium carboxymethyl cellulose, etc. The viscous agent may be used alone or two or more thereof may be used in combination. The cellulosic polymer is preferred and methyl cellulose is particularly preferred from the viewpoint of redispersibility.

The stabilizer is not particularly limited to the extent that it is pharmaceutically acceptable. Examples thereof include polyvinyl pyrrolidone, monoethanolamine, cyclodextrin, dextran, ascorbic acid, tocopherol, dibutylhydroxytoluene, a sulfite, and sodium edetate. A content thereof is preferably about 0.001 w/v% to about 1 w/v% relative to a total amount of the aqueous suspension liquid formulation.

The pH adjuster is not particularly limited to the extent that it is pharmaceutically acceptable. Examples thereof include an acid such as hydrochloric acid, acetic acid, boric acid, aminoethyl sulfonic acid, epsilon-aminocaproic acid, etc.; and an alkali such as sodium hydroxide, potassium hydroxide, borax, triethanolamine, monoethanolamine, sodium hydrogen carbonate, sodium carbonate, etc. A content thereof is, for example, 0 to about 20 w/v% relative to a total amount of the aqueous suspension liquid formulation.

In one embodiment of the present disclosure, the aqueous suspension liquid of the present disclosure may be mixed with the above-mentioned pH adjuster, if necessary, to achieve a pH of typically about 4 to about 8, preferably about 5.0 to about 8.0, more preferably about 6.0 to about 8.0, particularly preferably about 7.0 to about 8.0, and further preferably about 7.2 to about 7.8.

The buffer for use in the aqueous suspension liquid formulation of the present disclosure is not particularly limited to the extent that it is pharmaceutically acceptable. Examples thereof include a borate buffer, a phosphate buffer, a Tris buffer, a citrate buffer, a tartrate buffer, an acetate buffer, an amino acid buffer, etc., with a borate buffer or a phosphate buffer being preferred and a borate buffer being particularly preferred from the viewpoint of redispersibility or stability of the aqueous suspension liquid formulation.

A concentration of the buffer may be appropriately set within a range that can impart a desired buffering capacity to an aqueous liquid formulation and may be, for example, about 0.1 w/v% to about 10 w/v%, preferably about 1 w/v% to about 5 w/v%, and more preferably about 1 w/v% to about 3 w/v% from the viewpoint of improvement of redispersibility or stability.

The borate buffer is not particularly limited as long as it is pharmaceutically acceptable. For example, boric acid and/or a salt thereof may be used. The boric acid is not particularly limited as long as it is pharmaceutically acceptable. Examples thereof include orthoboric acid, metaboric acid, and tetraboric acid, etc. The salt of boric acid is not particularly limited as long as it is pharmaceutically acceptable. Examples thereof include a metal salt such as a sodium salt, a potassium salt, a calcium salt, a magnesium salt, an aluminum salt, etc.; and an organic amine salt such as triethylamine, triethanolamine, morpholine, piperazine, pyrrolidine, etc. The boric acid or a salt thereof may be used alone or two or more thereof may be used in combination. One suitable aspect of the borate buffer is a combination of boric acid and borax.

When boric acid and borax are used in combination, a ratio of the boric acid and the borax is not particularly limited and may be, for example, 10 to 300 parts by mass, preferably 10 to 250 parts by mass, more preferably 30 to 100 parts by mass, and particularly preferably 40 to 60 parts by mass of the borax per 100 parts by mass of the boric acid.

Specifically, the phosphate buffer may be phosphoric acid and/or a salt thereof. The salt of phosphoric acid is not particularly limited to the extent that it is pharmaceutically acceptable. Examples thereof include a dialkali metal salt of hydrogen phosphate such as disodium hydrogen phosphate, dipotassium hydrogen phosphate, etc.; an alkali metal salt of dihydrogen phosphate such as sodium dihydrogen phosphate, potassium dihydrogen phosphate, etc.; and a trialkali metal salt of phosphoric acid such as trisodium phosphate, tripotassium phosphate, etc. The salt of phosphoric acid may also be in a form of a solvate such as a hydrate, for example, disodium hydrogen phosphate may be in a form of a dodecahydrate, or sodium dihydrogen phosphate may be in a form of a dihydrate. One selected from the phosphoric acid and the salt thereof may be used alone or two or more thereof may be used in combination as the phosphate buffer. Among the phosphoric acid and the salt thereof, a phosphate is preferred, at least one of a dialkali metal salt of hydrogen phosphate and an alkali metal salt of dihydrogen phosphate is more preferred, and at least one of disodium hydrogen phosphate and sodium dihydrogen phosphate is particularly preferred.

The Tris buffer may be Tris (also known as tris-hydroxymethylaminomethane) and/or a salt thereof. The salt of Tris is not particularly limited as long as it is pharmaceutically acceptable. For example, a salt such as an acetate, a hydrochloride, a maleate, or a sulfonate may be used. One selected from the Tris and the salt thereof may be used alone or two or more thereof may be used in combination as the Tris buffer. In another embodiment, the Tris buffer may be specifically trometamol and/or a salt thereof. The salt of trometamol is not particularly limited to the extent that it is pharmaceutically acceptable. For example, an organic acid salt such as an acetate; an inorganic acid salt such as a hydrochloride and a sulfonate may be used. One selected from the trometamol and the salt thereof may be used alone or two or more thereof may be used in combination as the Tris buffer. Among the trometamol and the salt thereof, trometamol is preferred.

Specifically, the citrate buffer may be citric acid and/or a salt thereof. The salt of citric acid is not particularly limited to the extent that it is pharmaceutically acceptable. Examples thereof include an alkali metal salt such as a sodium salt and a potassium salt; an alkaline earth metal salt such as a calcium salt and a magnesium salt, etc. The salt of citric acid may also be in a form of a solvate such as a hydrate. One selected from the citric acid and the salt thereof may be used alone or two or more thereof may be used in combination as the citrate buffer. Among the citric acid and the salt thereof, a salt of citric acid is preferred, an alkali metal salt of citric acid is more preferred, and sodium citrate is particularly preferred.

Specifically, the tartrate buffer may be tartaric acid and/or a salt thereof. The salt of tartaric acid is not particularly limited to the extent that it is pharmaceutically acceptable. Examples thereof include an alkali metal salt such as a sodium salt and a potassium salt; an alkaline earth metal salt such as a calcium salt and a magnesium salt, etc. The salt of tartaric acid may also be in a form of a solvate such as a hydrate. One selected from the tartaric acid and the salt thereof may be used alone or two or more thereof may be used in combination as the tartrate buffer.

Specifically, the acetate buffer may be acetic acid and/or a salt thereof. The salt of acetic acid is not particularly limited to the extent that it is pharmaceutically acceptable. Examples thereof include an alkali metal salt such as a sodium salt and a potassium salt; an alkaline earth metal salt such as a calcium salt and a magnesium salt; and an ammonium salt, etc. The salt of acetic acid may also be in a form of a solvate such as a hydrate. One selected from the acetic acid and the salt thereof may be used alone or two or more thereof may be used in combination as the acetate buffer.

Specifically, the amino acid buffer may be an acidic amino acid and/or a salt thereof. Specific examples of the acidic amino acid include aspartic acid and glutamic acid. The salt of acidic amino acid is not particularly limited to the extent that it is pharmaceutically acceptable. For example, an alkali metal salt such as a sodium salt and a potassium salt may be used. One selected from the acidic amino acid and the salt thereof may be used alone or two or more thereof may be used in combination as the amino acid buffer.

The preservative is not particularly limited as long as it is pharmaceutically acceptable. Examples thereof include sorbic acid, potassium sorbate, a paraoxybenzoate ester such as methyl paraoxybenzoate, ethyl paraoxybenzoate, propyl paraoxybenzoate, butyl paraoxybenzoate, etc., a quaternary ammonium salt such as chlorhexidine gluconate, benzalkonium chloride, benzethonium chloride, cetylpyridinium chloride, etc., alkyl polyaminoethylglycine, chlorobutanol, polyquad, polyhexamethylene biguanide, chlorhexidine, etc. A content thereof can be appropriately varied according to the type and may be, for example, about 0.0001 w/v% to about 0.2 w/v% relative to a total amount of the aqueous suspension liquid formulation.

An eye drop can be prepared, for example, by dissolving or suspending the desired components as described above in an aqueous solvent such as sterile purified water, a saline solution, a buffer solution (e.g., phosphate buffer, citrate buffer, or acetate buffer, etc.) or a non-aqueous solvent such as a vegetable oil, for example, a cottonseed oil, a soybean oil, a sesame oil, or a peanut oil, etc.; adjusting the resulting solution or suspension to a predetermined osmotic pressure; and subjecting the resultant to a sterilization treatment such as filtration sterilization.

### (Container)

A container for containing the aqueous suspension liquid formulation of the present disclosure is not particularly limited, and include, for example, a glass container or a plastic container. The plastic container can be formed of any material such as polyester (polyethylene terephthalate, polyarylate), polycarbonate, polyethylene, or polypropylene, a mixture thereof, or a mixture with another material. A container for use in the present disclosure may or may not be those used in the medical field. In one embodiment, it can be formed of any material that can meet the "Standard for Plastic Containers for Eye Drops" or other equivalent standards in Japan.

The container to be used may have any shape, and any shape may typically be used as long as it is for an eye drop.

In a certain embodiment, the aqueous suspension liquid formulations of the present disclosure can be filled into any container for an eye drop commonly used in the medical field, for example, a polyethylene (preferably low-density polyethylene) container or a polypropylene container, preferably a colorless polypropylene container.

### (General technologies)

Molecular biological, biochemical, and microbiological procedures used herein are well known and common in the art, and are described in, for example, Sambrook J. et al. (1989). Molecular Cloning: A Laboratory Manual, Cold Spring Harbor and its 3rd Ed. (2001); Ausubel, F. M. (1987). Current Protocols in Molecular Biology, Greene Pub. Associates and Wiley-Interscience; Ausubel, F. M. (1989). Short Protocols in Molecular Biology: A Compendium of Methods from Current Protocols in Molecular Biology, Greene Pub. Associates and Wiley-Interscience; Innis, M. A. (1990). PCR Protocols: A Guide to Methods and Applications, Academic Press; Ausubel, F. M. (1992). Short Protocols in Molecular Biology: A Compendium of Methods from Current Protocols in Molecular Biology, Greene Pub. Associates; Ausubel, F. M. (1995). Short Protocols in Molecular Biology: A Compendium of Methods from Current Protocols in Molecular Biology, Greene Pub. Associates; Innis, M. A. et al. (1995). PCR Strategies, Academic Press; Ausubel, F. M. (1999). Short Protocols in Molecular Biology: A Compendium of Methods from Current Protocols in Molecular Biology, Wiley, and annual updates; Sninsky, J. J. et al. (1999). PCR Applications: Protocols for Functional Genomics, Academic Press, Gait, M. J. (1985). Oligonucleotide Synthesis: A Practical Approach, IRL Press; Gait, M. J. (1990). Oligonucleotide Synthesis: A Practical Approach, IRL Press; Eckstein, F. (1991). Oligonucleotides and Analogues: A Practical Approach, IRL Press; Adams, R. L. et al. (1992). The Biochemistry of the Nucleic Acids, Chapman & Hall; Shabarova, Z. et al. (1994). Advanced Organic Chemistry of Nucleic Acids, Weinheim; Blackburn, G. M. et al. (1996). Nucleic Acids in Chemistry and Biology, Oxford University Press; Hermanson, G. T.(I996). Bioconjugate Techniques, Academic Press, and Bessatsu Jikken Igaku "Experimental Methods for Gene Transfer & Expression Analysis", Yodosha, 1997, etc., each of which is incorporated herein by reference in its relevant parts (may be entirety).

References cited herein such as scientific literature, patents, and patent applications are incorporated herein by reference in its entirety as if specifically set forth herein.

The present disclosure has been described with reference to preferred embodiments for ease of understanding. Hereinafter, the present disclosure will be described with reference to Examples, but the above description and Examples below are provided for illustrative purposes only and are not intended to limit the present disclosure. Accordingly, the scope of the present disclosure is not limited to embodiments or Examples specifically described herein, but is limited only by claims.

### [Example]

### (Test Example 1: Change in redispersibility of drug substance by heat treatment temperature)

### Preparation of suspension liquid

Base solutions were prepared according to the compositions shown in Tables 1 and 2 and Compound (1) was added to each of the base solutions and dispersed therein with stirring. Thus, suspension liquids were obtained. Tyloxapol manufactured by AMRI Rensselaer (Curia Global, Inc.) was used. The Compound (1) was in the form of the above-described type I crystal.

### Containerization

Each of the thus-prepared suspension liquids was collected in 5 mL portions with stirring by a stirrer and filled into eye drop containers. Each of the eye drop container was a colorless container made of polyethylene (container used for GATIFLO eye drop 0.3% (manufactured and sold by Senju Pharmaceutical Co., Ltd.)).

### Evaluation of redispersibility

Evaluation method by inversion operation: Suspended particles in the suspension liquid filled in the eye drop container were confirmed to have completely settled. The inversion operation (i.e., holding the container in the hand and turning it up and down) was repeated until a precipitate disappeared from a bottom surface and a wall of the eye drop container and redispersed. Note that, the container was turned 180° vertically and then turned 180° again to stand upright at a rate of 1 second/inversion, which was defined as one inversion. The number of the inversions required for redispersion was counted. The test was performed on three samples per formulation and an average number of inversions was calculated.

Evaluation method by shaking operation: Suspended particles in the suspension liquid filled in the eye drop container were confirmed to have completely settled. Five shakes were determined as one set and shake was repeated until a precipitate disappeared from a bottom surface and a wall of the eye drop container and redispersed. Note that, one shake was defined as follows: the container was shaken down 10 to 5 cm vertically and then shaken up to its original position. This action was performed at a rate of 1.1 seconds/set. The number of sets required for redispersion was counted and the number of shakes was calculated.

### Measurement of particle size

After shaking a sample until a precipitate redispersed, approximately 1 mL of the sample was added dropwise to a dispersion tank for a laser diffraction particle size analyzer (SALD-2300). After sonication for 2 minutes, particle size distribution was measured and D10, D50, and D90 values were determined as a particle size.

### Result

The results are shown in Tables 1 and 2. The aqueous suspension liquid formulations in which the Compound (1) which had been heat-treated at 150 to 170 °C was dispersed showed good redispersibility.

**[Table 1]**

| | Component | | Reference Example 1 | Example 1 | Example 2 | Example 3 | Example 4 | Comparative Example 1 | Comparative Example 2 |
|---|---|---|---|---|---|---|---|---|---|
| Composition | Compound (1) | Temperature ^{*1} | Untreated | 100°C | 150°C | 160°C | 170°C | 180°C | 200°C |
| | | Amount | 0.3 g | 0.3 g | 0.3 g | 0.3 g | 0.3 g | 0.3 g | 0.3 g |
| | Boric acid | | 2.0 g | 2.0 g | 2.0 g | 2.0 g | 2.0 g | 2.0 g | 2.0 g |
| | Borax | | 0.8 g | 0.8 g | 0.8 g | 0.8 g | 0.8 g | 0.8 g | 0.8 g |
| | Tyloxapol | | 0.04 g | 0.04 g | 0.04 g | 0.04 g | 0.04 g | 0.04 g | 0.04 g |
| | Purified water | | q.s. | q.s. | q.s. | q.s. | q.s. | q.s. | q.s. |
| | Total amount | | 100 mL | 100 mL | 100 mL | 100 mL | 100 mL | 100 mL | 100 mL |
| | pH | | 7.1 | 7.1 | 7.1 | 7.1 | 7.1 | 7.1 | 7.1 |
| Result | Number of inversion operations | | 50.0 | 52.3 | 40.7 | 40.7^{*3} | 39.7 | 63.3 | 65.7 |
| | Change rate ^{*2} | | - | 104.7% | 81.3% | 81.3% | 79.3% | 126.7% | 131.3% |

| | | | | | | | | | |
|---|---|---|---|---|---|---|---|---|---|
| *1 Treated at the predetermined temperature for 2 hours *2 Means a change rate of the number of inversion operations relative to that of Reference Example 1 *3 The number of shakes was 16.7 | | | | | | | | | |

**[Table 2]**

| | Component | | Reference Example 2 | Example 5 | Example 6 | Comparative Example 3 |
|---|---|---|---|---|---|---|
| Composition | Compound (1) | Temperature ^{*4} | Untreated | 160°C | 170°C | 200°C |
| | | Amount | 1.0 g | 1.0 g | 1.0 g | 1.0 g |
| | Boric acid | | 1.64 g | 1.64 g | 1.64 g | 1.64 g |
| | Borax | | 0.54 g | 0.54 g | 0.54 g | 0.54 g |
| | Tyloxapol | | 0.05 g | 0.05 g | 0.05 g | 0.05 g |
| | Purified water | | q.s. | q.s. | q.s. | q.s. |
| | Total amount | | 100 mL | 100 mL | 100 mL | 100 mL |
| | pH | | 7.5 | 7.5 | 7.5 | 7.5 |
| Result | Number of shakes | | 36.7 | 25.0 | 23.3^{*5} | 43.3 |
| | Particle size (*µ*m) | D10 | 0.9 | 1.2 | 1.2 | 1.7 |
| | | D50 | 1.8 | 2.9 | 3.0 | 4.6 |
| | | D90 | 3.4 | 5.8 | 5.8 | 9.3 |

| | | | | | | |
|---|---|---|---|---|---|---|
| *4 Treated at the predetermined temperature for 2 hours *5 The number of inversion operations was 45.7 | | | | | | |

### (Test Example 2: Heat treatment time)

### Preparation of suspension liquid

A base solution was prepared according to the composition shown in Table 3, to which Compound (1) was added and stirred to be dispersed therein. Thus, a suspension liquid was obtained.

### Containerization

The suspension liquid was filled into containers in the same manner as in Test Example 1.

### Evaluation of redispersibility

The suspension liquid was evaluated for redispersibility in the same manner as in Test Example 1.

### Result

The results are shown in Table 3. The aqueous suspension liquid formulations in which the Compound (1) which had been heat-treated for 30 minutes to 5 hours was dispersed showed good redispersibility.

**[Table 3]**

| | Component | | Example 7 | Example 3 | Example 8 |
|---|---|---|---|---|---|
| Composition | Compound (1) | Temperature | 160°C | 160°C | 160°C |
| | | Time | 30 min | 2 hr | 5 hr |
| | | Amount | 0.3 g | 0.3 g | 0.3 g |
| | Boric acid | | 2.0 g | 2.0 g | 2.0 g |
| | Borax | | 0.8 g | 0.8 g | 0.8 g |
| | Tyloxapol | | 0.04 g | 0.04 g | 0.04 g |
| | Purified water | | q.s. | q.s. | q.s. |
| | Total amount | | 100 mL | 100 mL | 100 mL |
| Result | Number of inversion operations | | 40.7 | 40.7 | 41.0 |

### (Test Example 3: Change in redispersibility caused by different surfactants)

### Preparation of suspension liquid

A base solution was prepared according to the composition shown in Table 4, to which Compound (1) was added and stirred to be dispersed therein. Thus, a suspension liquid was obtained. Tyloxapol from AMRI Rensselaer (Curia Global, Inc.) was used. Polysorbate 80 manufactured by NOF CORPORATION, polyethylene glycol monostearate 40 (MYS-40) manufactured by NIPPON SURFACTANT INDUSTRIES CO., LTD., and a polyoxyethylene hydrogenated castor oil 60 manufactured by NIPPON SURFACTANT INDUSTRIES CO., LTD. were used. The Compound (1) was in the form of the above-described type I crystal.

### Containerization

The suspension liquid was filled into containers in the same manner as in Test Example 1.

### Evaluation of redispersibility

The suspension liquid was evaluated for redispersibility in the same manner as in Test Example 1.

### Result

The results are shown in Table 4. The aqueous suspension liquid formulations in which the Compound (1) which had been heat-treated at 160 °C was dispersed showed good redispersibility in the presence of any noninoic surfactant.

**[Table 4]**

| | Component | | Reference Example 3 | Example 9 | Comparative Example 4 | Reference Example 4 | Example 10 | Comparative Example 5 | Reference Example 5 | Example 11 | Comparative Example 6 |
|---|---|---|---|---|---|---|---|---|---|---|---|
| Composition | Compound (1) | Temperature ^{*6} | Untreated | 160°C | 200°C | Untreated | 160°C | 200°C | Untreated | 160°C | 200°C |
| | | Amount | 1.0 g | 1.0 g | 1.0 g | 1.0 g | 1.0 g | 1.0 g | 1.0 g | 1.0 g | 1.0 g |
| | Boric acid | | 1.64 g | 1.64 g | 1.64 g | 1.64 g | 1.64 g | 1.64 g | 1.64 g | 1.64 g | 1.64 g |
| | Borax | | 0.54 g | 0.54 g | 0.54 g | 0.54 g | 0.54 g | 0.54 g | 0.54 g | 0.54 g | 0.54 g |
| | Polysorbate 80 | | 0.05 g | 0.05 g | 0.05 g | - | - | - | - | - | - |
| | MYS-40 | | - | - | - | 0.05 g | 0.05 g | 0.05 g | - | - | - |
| | HCO-60 | | - | - | - | - | - | - | 0.05 g | 0.05 g | 0.05 g |
| | Purified water | | q.s. | q.s. | q.s. | q.s. | q.s. | q.s. | q.s. | q.s. | q.s. |
| | Total amount | | 100 mL | 100 mL | 100 mL | 100 mL | 100 mL | 100 mL | 100 mL | 100 mL | 100 mL |
| | pH | | 7.5 | 7.5 | 7.5 | 7.5 | 7.5 | 7.5 | 7.5 | 7.5 | 7.5 |
| Result | Number of shakes | | 65.0 | 28.3 | 38.3 | 71.7 | 28.3 | 38.3 | 80.0 | 33.3 | 45.0 |

| | | | | | | | | | | | |
|---|---|---|---|---|---|---|---|---|---|---|---|
| *6 Treated at the predetermined temperature for 2 hours | | | | | | | | | | | |

### (Test Example 4: Concentration of surfactant)

### Preparation of suspension liquid

A base solution was prepared according to the composition shown in Table 5, to which Compound (1) was added and stirred to be dispersed therein. Thus, a suspension liquid was obtained. Tyloxapol from AMRI Rensselaer (Curia Global, Inc.) was used. The Compound (1) was in the form of the above-described type I crystal.

### Containerization

The suspension liquid was filled into containers in the same manner as in Test Example 1.

### Evaluation of redispersibility

The suspension liquid was evaluated for redispersibility in the same manner as in Test Example 1.

### Result

The results are shown in Table 5. The aqueous suspension liquid formulations which included tyloxapol at a concentration in a range of 0.01% to 0.04% showed good redispersibility.

**[Table 5]**

| | Component | | Example 3 | Example 12 |
|---|---|---|---|---|
| Composition | Compound (1) | Temperature^{*7} | 160°C | 160°C |
| | | Amount | 0.3 g | 0.3 g |
| | Boric acid | | 2.0 g | 2.0 g |
| | Borax | | 0.8 g | 0.8 g |
| | Tyloxapol | | 0.04 g | 0.01 g |
| | Purified water | | q.s. | q.s. |
| | Total amount | | 100 mL | 100 mL |
| Result | Number of inversion operations | | 40.7 | 41.0 |

| | | | | |
|---|---|---|---|---|
| *7 Treated at the predetermined temperature for 2 hours | | | | |

### (Test Example 5: Change in redispersibility caused by different sterilization methods)

### Preparation of suspension liquid

A base solution was prepared according to the composition shown in Table 6, to which Compound (1) was added and stirred to be dispersed therein. Thus, a suspension liquid was obtained. Tyloxapol from AMRI Rensselaer (Curia Global, Inc.) was used. The Compound (1) was in the form of the above-described type I crystal. The Compound (1) which had been irradiated with about 25 kGy of electron rays was used in Comparative Example 7, and the Compound (1) which had been irradiated with about 25 kGy of gamma rays was used in Example 13. The Compound (1) which had been sterilized with a dry heat treatment at 160 °C for 2 hours was used in Example 14.

### Containerization

The suspension liquid was filled into containers in the same manner as in Test Example 1.

### Evaluation of redispersibility

The suspension liquid was evaluated for redispersibility in the same manner as in Test Example 1.

### Result

The results are shown in Table 6. The aqueous suspension liquid formulation in which the Compound (1) which had been treated with gamma rays was dispersed showed good redispersibility.

**[Table 6]**

| | Component | | Reference Example 6 | Comparative Example 7 | Example 13 | Example 14 |
|---|---|---|---|---|---|---|
| Composition | Compound (1) | Sterilization method | Untreated | Electron ray | Gamma ray | 160°C • 2 hr |
| | | Amount | 0.3 g | 0.3 g | 0.3 g | 0.3 g |
| | Boric acid | | 1.2 g | 1.2 g | 1.2 g | 1.2 g |
| | Borax | | 0.4 g | 0.4 g | 0.4 g | 0.4 g |
| | Tyloxapol | | 0.05 g | 0.05 g | 0.05 g | 0.05 g |
| | Purified water | | q.s. | q.s. | q.s. | q.s. |
| | Total amount | | 100 mL | 100 mL | 100 mL | 100 mL |
| Result | Number of inversion operations | | 53.3 | 53.7 | 40.7 | 41.3 |

### (Test example: Stability of formulation using dry-heated drug substance)

### Preparation of suspension liquid

Suspension liquids were obtained according to compositions shown in Table 7. Tyloxapol from AMRI Rensselaer (Curia Global, Inc.) was used. The Compound (1) was in the form of the above-described type I crystal.

**[Table 7]**

| Component | | Example 15 | Example 16 |
|---|---|---|---|
| Compound (1) | Temperature | 160°C | 160°C |
| | Time | 2 hr | 2 hr |
| | Amount | 0.03 g | 1 g |
| Boric acid | | 1.59 g | 1.59 g |
| Borax | | 0.74 g | 0.74 g |
| Tyloxapol | | 0.02 g | 0.2 g |
| Purified water | | q.s. | q.s. |
| Total amount | | 100 mL | 100 mL |
| pH | | 7.7 | 7.7 |

### Containerization

Each of the suspension liquids was filled into containers in the same manner as in Test Example 1.

### Storage

Each of the suspension liquids was stored under the following conditions.

Storage conditions: 60 ± 2 °C (average: 60.1 °C), humidity without artificial control.

Storage period: at start, 2 weeks, 4 weeks

### Measurement of pH

A pH was measured according to the pH measurement method in the general test method of the Japanese Pharmacopoeia. That is, the pH was measured using a pH meter with a glass electrode.

### Measurement of osmotic pressure

Osmotic pressure was measured by the osmotic pressure measurement method (osmolarity measurement method) in the general test method of the Japanese Pharmacopoeia.

### Measurement of content and impurity

In Example 15, a sample solution was prepared by diluting the suspension liquid 2-fold with acetonitrile. In Example 16, 2 mL of the suspension liquid was accurately weighed and a mobile phase B was added thereto to give a volume of exactly 20 mL and dissolved thereinto, which was used as a sample solution. Separately, about 20 mg of Compound (1) was precisely weighed and a mobile phase B was added thereto to give a volume of exactly 20 mL and dissolved thereinto, which was used as a standard solution A. Two milliliters of this solution was accurately weighed and a mobile phase B was added thereto to give a volume of exactly 20 mL and dissolved thereinto, which was used as a standard solution B. The standard solution B was used in Example 15 and the standard solution A was used in Example 16. Ten microliters of each of the sample and standard solutions were accurately taken and tested by liquid chromatography under the following conditions.

### Test conditions

Detector: UV absorption spectrophotometer (measurement wavelength: 250 nm)
Column: A commercially available column made of a stainless steel tube with an inner diameter of 4.6 mm and a length of 250 mm filled with 5 µm octadecylsilylated silica gel for liquid chromatography was used.
Column temperature: Constant temperature around 40 °C
Mobile phase A: 1.79 g of disodium hydrogen phosphate dodecahydrate was dissolved in 1000 mL of water. Phosphoric acid is added to the resulting solution to adjust the pH to 6.5. Then, 350 mL of acetonitrile was added to 650 mL of the solution and mixed together.
Mobile phase B: Acetonitrile/water mixed solution (80:20)
Pumping of Mobile phase: A mixing ratio of the mobile phases A and B was controlled according to the linear concentration gradient shown in the Table below.

### Measurement of particle size

The suspension liquid was measured for a particle size in the same manner as in Test Example 1.

### Result

The results (n = 3) are shown in Table 8. Storage stability for 4 weeks was shown in each case.

**[Table 8]**

| Item | | Example 15 | | | Example 16 | | |
|---|---|---|---|---|---|---|---|
| | | At start | 2 weeks | 4 weeks | At start | 2 weeks | 4 weeks |
| Osmotic pressure (mOsmol/kg) | | 293 | 298 | 300 | 296 | 301 | 304 |
| pH | | 7.7 | 7.7 | 7.7 | 7.7 | 7.7 | 7.7 |
| Content (%) | | 97.0 | 98.9 | 100.1 | 99.8 | 101.5 | 99.9 |

| Impurity content (%) | | Below limit of quantification (0.1) | Below limit of quantification (0.1) | Below limit of quantification (0.1) | Below limit of quantification (0.1) | Below limit of quantification (0.1) | Below limit of quantification (0.1) |
|---|---|---|---|---|---|---|---|
| Particle size (*µ*m) | D₁₀ | 1.2 | 1.2 | 1.2 | 1.2 | 1.2 | 1.2 |
| | D₅₀ | 2.5 | 2.5 | 2.5 | 2.6 | 2.6 | 2.5 |
| | D₉₀ | 4.9 | 4.8 | 4.7 | 5.0 | 4.8 | 4.8 |
| | D_{99. 999} | 10.4 | 10.3 | 10.3 | 10.4 | 10.4 | 10.4 |

### (Notes)

Although the present disclosure has been illustrated with reference to preferred embodiments of the present disclosure, it is understood that the scope of the present disclosure should be construed only by the claims. It is to be understood that the patents, patent applications, and other references cited herein should be incorporated herein by reference in its entirety as if the contents themselves are specifically set forth herein. This application claims priority to Japanese Patent Application No. 2022-156060 filed on September 29, 2022, with the Japan Patent Office, the content of which is incorporated herein by reference in their entirety.

### [Industrial Applicability]

The present disclosure is available in the fields of medicine, pharmaceuticals, healthcare, biology, biochemistry, etc.

## Claims

1. An aqueous suspension liquid formulation comprising
(E)-2-(7-trifluoromethylchroman-4-ylidene)-N-(7-hydroxy-5,6,7,8-tetrahydronaphthalen-1-yl)acetamide or a pharmaceutically acceptable salt or solvate thereof which has been subjected to a heat treatment.

2. The aqueous suspension liquid formulation according to claim 1, wherein the heat treatment is a treatment in a sterilization step.

3. The aqueous suspension liquid formulation according to any one of claim 1 or 2, further comprising a nonionic surfactant.

4. The aqueous suspension liquid formulation according to any one of claims 1 to 3, wherein the heat treatment comprises a heat treatment at a temperature of less than about 180 °C.

5. The aqueous suspension liquid formulation according to any one of claims 1 to 4, wherein the heat treatment comprises a heat treatment at about 100 °C to about 175 °C.

6. The aqueous suspension liquid formulation according to any one of claims 1 to 5, wherein the heat treatment comprises a heat treatment at about 150 °C to about 170 °C.

7. The aqueous suspension liquid formulation according to any one of claims 1 to 6, wherein the heat treatment is performed for about 30 minutes to about 5 hours.

8. The aqueous suspension liquid formulation according to any one of claims 3 to 7, wherein the nonionic surfactant has a concentration of about 0.0001 w/v% to about 1 w/v% in the aqueous suspension liquid formulation.

9. The aqueous suspension liquid formulation according to any one of claims 3 to 8, wherein the nonionic surfactant is at least one selected from the group consisting of tyloxapol, polysorbate, polyethylene glycol monostearate, and a polyoxyethylene hydrogenated castor oil.

10. The aqueous suspension liquid formulation according to any one of claims 1 to 9, wherein the (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof has a concentration of about 0.01 w/v% to about 5 w/v% in the aqueous suspension liquid formulation.

11. The aqueous suspension liquid formulation according to any one of claims 1 to 10, wherein the (E)-2-(7-trifluoromethylchroman-4-ylidene)-N-(7-hydroxy-5,6,7,8-tetrahydronaphthalen-1-yl)acetamide is (E)-2-(7-trifluoromethylchroman-4-ylidene)-N-((7R)-7-hydroxy-5,6,7,8-tetrahydronaphthalen-1-yl)acetamide.

12. An aqueous suspension liquid formulation, comprising
(E)-2-(7-trifluoromethylchroman-4-ylidene)-N-(7-hydroxy-5,6,7,8-tetrahydronaphthalen-1-yl)acetamide or a pharmaceutically acceptable salt or solvate thereof which has been subjected to a heat treatment, the (E)-2-(7-trifluoromethylchroman-4-ylidene)-N-(7-hydroxy-5,6,7,8-tetrahydronaphthalen-1-yl)acetamide or the pharmaceutically acceptable salt or solvate thereof having improved redispersibility in the aqueous suspension liquid formulation.

13. The aqueous suspension liquid formulation according to claim 12, wherein the improved redispersibility is a property of redispersing suspended particles in less than about 35 shakes as evaluated by a shaking operation.

14. The aqueous suspension liquid formulation according to claim 12 or 13, wherein the improved redispersibility is a property of redispersing suspended particles in less than about 50 inversions as evaluated by an inversion operation.

15. An aqueous suspension liquid formulation, comprising
(E)-2-(7-trifluoromethylchroman-4-ylidene)-N-(7-hydroxy-5,6,7,8-tetrahydronaphthalen-1-yl)acetamide or a pharmaceutically acceptable salt or solvate thereof which has been subjected to a heat treatment, the (E)-2-(7-trifluoromethylchroman-4-ylidene)-N-(7-hydroxy-5,6,7,8-tetrahydronaphthalen-1-yl)acetamide or the pharmaceutically acceptable salt or solvate thereof having an average particle size (D50) of about 1 µm to about 10 µm in the aqueous suspension liquid formulation.

16. A method for producing an aqueous suspension liquid formulation, the method comprising:
heat-treating (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof; and
mixing the thus-heat-treated (E)-2-(7-trifluoromethylchroman-4-ylidene)-N-(7-hydroxy-5,6,7,8-tetrahydronaphthalen-1-yl)acetamide or the pharmaceutically acceptable salt or solvate thereof with a solvent.

17. (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof which has been subjected to a heat treatment.
